# EUROPEAN PATENT APPLICATION

(11) **EP 2 471 553 A1**
(43) Date of publication of application: **04.07.2012**
(21) Application number: 09846341.7
(22) Date of filing: 26.06.2009
(51) Int. Cl.: A61K 39/245, A61P 31/22

(54) **AN IMMUNOGENIC COMPOSITION COMPRISING PEPTIDES DERIVED FROM CYTOMEGALOVIRUS AND THE USE THEREOF**

(71) Applicant: Vectorite Biomedica Inc., Taipei, Taiwan (TW); Beijing Ctl Biomedica Inc., Beijing 100039 (CN)
(72) Inventor: LIANG, Yin, Taipei (TW); HUANG, Yu-Ju, Taipei (TW); YANG, Fu-Hung, Taipei (TW)
(74) Representative: Becker Kurig Straus
(86) International application number: PCT/CN2009/000718
(87) International publication number: WO 2010/148541

(57) **Abstract**

The present invention provides (poly)peptides, which are recognized by human cytomegalovirus (CMV)-specific immune cells. The present invention further provides a combination of multiple CMV (poly)peptides, comprising at least two different groups of (poly)peptides according to the invention as well as conjugates, comprising said (poly)peptides and/or immune adjuvants thereof. Furthermore, this invention provides mixtures, comprising said (poly)peptides and/or immune cells (20) thereof, which are used to generate CMV-specific immune effector cells (31) with high sensitivity and specificity. In addition, the present invention provides a preparation method of CMV-specific immune effector cells (31), by using said (poly)peptides, adjuvants, immune cells (20) and/or mixtures thereof to generate anti-CMV immune response.

## Description

The present invention relates to an anti-herpes virus vaccine, particularly for producing the antigen composition of anti-herpes virus of immune effector cells. The present invention also relates to the use of the above antigen composition and the method for activating immune cells.

Herpes virus belongs to *Herpesviridae.* As currently known, eight kinds of viruses cause diseases; these viruses are called human herpes viruses (HHV), including: (1) human herpes cirus-1 (HHV-1), also known as herpes simplex virus-1 (HSV-1); (2) human herpes virus-2 (HHV-2), also known as herpes simplex virus-2 (HSV-2); (3) human herpes virus-3 (HHV-3), also known as Varicella Zoster Virus (VZV), which causes chicken pox and herpes zoster and is also called varicella virus; (4) human herpes virus-4 (HHV-4), also known as Epstein-Barr Virus (EBV), which is a lymphocytic virus, causes Burkitt's lymphoma and nasopharyngeal carcinoma; (5) human herpes virus-5 (HHV-5), also known as cytomegalovirus (CMV); (6) human herpes virus-6 (HHV-6), also known as roseolovirus, which causes the sixth syndrone including roseola infantum and exanthema subitum; (7) human herpes virus-7 (HHV-7), related to HHV-6 and causing similar symptoms; and (8) human herpes virus-8 (HHV-8), which is Genus Rhadinovirus, referred to as kSHV, and could be found in Kaposi's sarcoma.

Current management of herpes virus infections in transplant recipients includes prophylaxis or preemptive treatment with antiviral agents. Taking Cytomegalovirus (CMV) as an example, for the former is positive in culture test or expresses virus active replication in test, to heal beforehand without any symptoms. The latter is aimed at serum positive for healing beforehand without any symptoms. There are several antiviral drugs available against CMV replication, including ganciclovir, foscarnet, and cidofovir. (Gandhi MK, et al., Lancet Infect. Dis., 2004, 4:725-38) Significant progress has been made in the control of CMV infection in transplant recipients with these agents.

However, these pharmacological strategies have limitations, e.g., drug toxicities, development of resistance, poor oral bioavailability, and low potency. Mortality remains high even though patients are treated with antiviral agents, especially when therapy is not initiated early in the course. (Gandhi MK, et al., Lancet Infect. Dis., 2004, 4:725-38; Ison MG, et al., Clin. Chest Med., 2005, 26:691-705; Gandhi MK, et al., Blood Rev., 2003, 17:259-64) In addition, resistance to antiviral agents emerges in patients after prolonged exposure to this agent (Ison MG, *et al., supra*; Biron KK., Antiviral Res., 2006, 71: 154-63).

Existing immune-therapies for herpes virus infected diseases use herpes virus antigen sources based on live viruses, herpes virus-infected cells, herpes virus gene expression vectors, or synthetic herpes virus proteins or peptides to prime antigen-presenting cells for the activation of herpes virus-specific immune response. The use of viruses or virus-infected cells as antigen source has potential risk of virus contamination and infection. The use of full-length herpes virus genes or proteins as antigen source has potential risk of induction of immune suppression or tolerance.

Given that the aforesaid drawbacks of the prior art such as virus-infection and immune tolerance, the objective of the present invention provides an immune therapy for herpes virus, which does not produce problems as which are caused by using live viruses, herpes virus-infected cells or full-length herpes virus proteins.

Therefore, in one aspect, the present invention provides an immunogenic composition containing peptides derived from CMV, which comprises: (a) at least one peptide fragment selected from CMV pp65-derived and CMV IE-1-derived peptide fragments; and (b) one or more peptide fragment(s) selected from the group consisting of CMV VGLB, CMV VPAP and CMVp 100 polypeptide-derived peptide fragments.

In a second aspect, the present invention provides an immunogenic composition containing peptides derived from CMV, which comprises: (a) at least one peptide pool selected from CMV pp65 and CMV IE-1 polypeptides-derived peptide pools; and (b) one or more peptide pool(s) selected from the group consisting of CMV VGLB, CMV VPAP and CMVp100 polypeptide-derived peptide pool.

In a preferred embodiment of the immunogenic composition of the present invention, CMV pp65 has a sequence essentially identical to the sequence as set forth in SEQ ID NO: 1; CMV IE-1 has a sequence essentially identical to the sequence as set forth in SEQ ID NO: 2; CMV VGLB has a sequence essentially identical to the sequence as set forth in SEQ ID NO: 3; CMV VPAP has a sequence essentially identical to the sequence as set forth in SEQ ID NO: 4; and CMV p100 has a sequence essentially identical to the sequence as set forth in SEQ ID NO: 5.

Preferably, said immunogenic composition comprises CMV pp65, CMV IE-1, CMV VGLB, CMV VPAP and CMV p100 polypeptide-derived peptide pools.

In a third aspect, the present invention provides use of the immunogenic composition as in any of the aforesaid first to second aspects for *ex vivo* inducing immune response for herpes virus.

In a fourth aspect, the present invention provides use of the immunogenic composition as in any of the aforesaid first to second aspects for manufacturing medicament for treating herpes virus infection diseases.

In a fifth aspect, the present invention provides a method for *ex vivo* activating immune cell, comprising steps of: mixing an immune cell and an immunogenic composition, as in any of the aforesaid first to second aspects, to form a mixed culture; then incubating the mixed culture in a suitable medium to obtain the activated immune cell.

In an sixth aspect, the present invention provides a method for *ex vivo* inducing production of immune effector cells, comprising steps of: providing an activated immune cell as in any of the aforesaid first to second aspects and a lymphocyte; then co-incubating the activated immune cell and lymphocyte in a suitable medium to obtain the immune effector cell.

The objective of the present invention proves the immunogenic composition could activate anti-CMV immune response effectively, and the peptide pool has non-live virus, non-CMV infected cell, therefore not producing potential risk such as infection, immune inhibition and immune tolerance. Moreover, the method according to the present invention could activate immune cells effectively, and further activate lymphocytes, and produce the immune cells having activity against CMV

### IN THE DRAWINGS

Fig. 1 is a schematic flow chart diagram illustrating the method of the present invention;
Fig. 2 illustrates the diagram of CMV polypeptide-derived peptide pool, with pp65 as the polypeptide;
Fig. 3 illustrates the flow chart of production of activated lymphocytes, which are activated by dendritic cells;
Fig. 4 illustrates the effect of various CMV proteins-derived peptide pools and their combinations on inducing proliferation of PBMC, wherein panel A illustrates the result of CFSE staining analysis, and panel B illustrates the result of intracellular cytokine staining analysis;
Fig. 5 illustrates the effect of various CMV proteins-derived peptide pools and their combinations on inducing immune response of PBMC, wherein panel A and B illustrate the results of various donators' PBMCs;
Fig. 6 illustrates the effect of various CMV proteins-derived peptide pools and their combination on cell proliferations of populations of CD4⁺ and CD8⁺ cells in PBMCs;
Fig. 7, in panels A and B, illustrates the cytokine secretion of PBMCs of various origins stimulated by various CMV proteins-derived peptide pools and their combinations;
Fig.8 illustrates the result of cellular cytokine staining analysis of CMV-specific immune effector cells, which were non-adherent PBMCs stimulated by DCs pulsed with the mixed pool of 5 CMV pentadecapeptides ; and
Fig.9 is the bar chart of the result of Fig. 8; panel A illustrates expression of IL-2 of each group, and panel B illustrates expression of CD107a of each group.

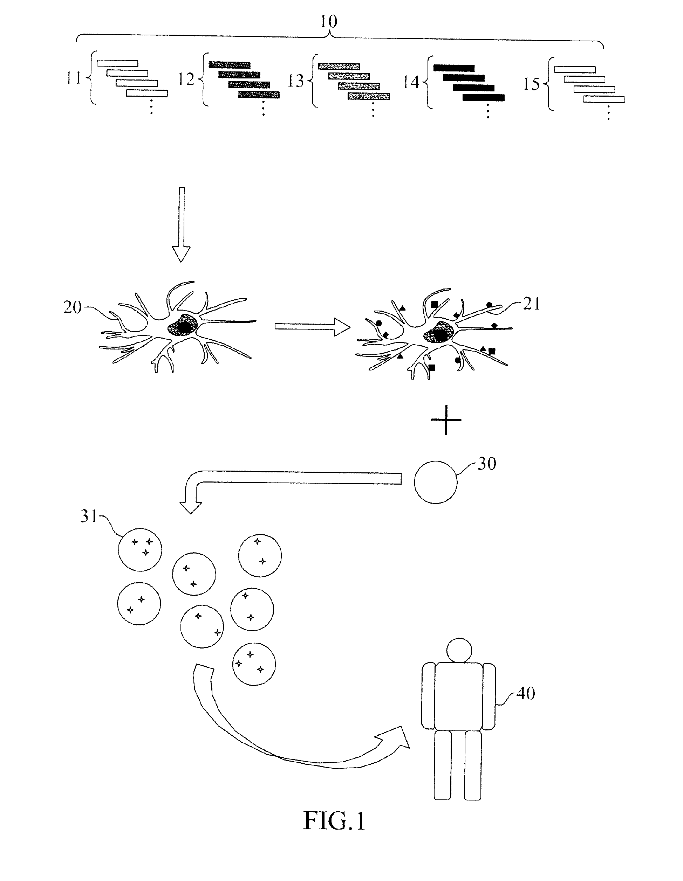

The applicants of the present invention develop an immunogenic composition and a method for immune effector cells against herpes virus. With reference to Fig. 1, the method of the present invention involves the following aspects. First, using various immunogenic compositions (10) containing peptides derived from CMV peptides to stimulate immune cells (20), wherein the immunogenic composition (10), for example, includes peptide pools derived from CMV pp65, CMV IE-1, CMV VGLB, CMV VPAP and CMV p100 polypeptides (11) (12) (13) (14) (15) to obtain activated immune cells (21) with capability of activating lymphocytes (30) such as T lymphocytes. Second, activated immune cells and lymphocytes are co-incubated to obtain immune effector cells (31). Then, the immune effector cells are further implanted into individuals (40) to achieve the purpose of preventing and treating herpes virus related diseases.

Applicants discover the mixed pools of two, three, four or five CMV peptide pools, compared with only single or double CMV protein-derived peptide pools, are more effective in activating anti-CMV immune response; especially CMV specific CD8⁺ and CD4⁺ T lymphocytes are proved activated to generate high effector functions. Such reveals that CMV immunogenic composition and immune treatment of the present invention could be used to prevent and treat herpes virus related diseases.

Therefore, in one aspect, the present invention provides an immunogenic composition containing peptides derived from CMV, which comprises: (a) at least one peptide selected from CMV pp65 and CMV IE-1 polypeptides-derived peptide; and (b) one or more peptide pool(s) selected from the group consisting of peptide pools of CMV VGLB, CMV VPAP and CMVp100 polypeptides-derived peptide.

In another aspect, the present invention provides an immunogenic composition containing peptides derived from CMV, which comprises: (a) at least one peptide pool selected from CMV pp65 and CMV IE-1 polypeptides-derived peptide pools; and (b) one or more peptide pool(s) selected from the group consisting of CMV VGLB, CMV VPAP and CMVp100 polypeptides-derived peptide pools.

CMV pp65 is a CMV structural protein, which is referred to as HCMV phosphorylated matrix protein, and includes the following characteristics: (1) Name: pp65, 65 kDa lower matrix phosphoprotein, i.e. Tegument protein UL83; (2) Organism: Human cytomegalovirus, belonging to Human herpesvirus-5 (HHV-5); (3) Sequence length: 561 amino acids; and (4) Function: forming part of the matrix of the HCMV virion. In a preferred embodiment, the sequence is as set forth in serial number Swiss-Prot: P06725, NCBI Accession NO: NP_040018 or SEQ ID NO: 1.

CMV IE-1 is a CMV major immediate early protein-1, which is a transcription regulatory protein, and includes the following characteristics: (1) Name: IE-1, UL123; (2) Organism: Human cytomegalovirus, belonging to Human herpesvirus-5 (HHV-5); (3) Sequence length: 491amino acids; and (4) Function: Immediate-early transcriptional regulator. The IE1 protein augments the activation of the El.7 promoter by EI2. In a preferred embodiment, the sequence is as set forth in serial number Swiss-Prot: P13202 sequence, NCB I Accession NO: NP_040060 or SEQ ID NO: 2.

CMV VGLB is a CMV viral envelope glycoprotein, and includes the following characteristics: (1) Name: Glycoprotein B (gB), also named Glycoprotein GP55, and UL55; (2) Organism: Human cytomegalovirus, belonging to Human herpesvirus-5 (HHV-5); (3) Sequence length: 907 amino acids; and (4) Function: Type 1 membrane protein and also as a viral ligand for CD209/DC-SIGN to allow capturing of viral particles by dendritic cells (DCs) and subsequently transferring viruses to permissive cells. In a preferred embodiment, the sequence is as set forth in serial number NCBI Accession NO: YP-081514 or SEQ ID NO: 3.

CMV VPAP is a CMV DNA polymerase processivity factor, which is subunit of HCMV polymerase protein, and includes the following characteristics: (1) Name: DNA polymerase processivity factor, also named Polymerase accessory protein (PAP), and UL44; (2) Organism: Human cytomegalovirus, belonging to Human herpesvirus-5 (HHV-5); (3) Sequence length: 433 amino acids; and (4) Function: accessory subunit of the DNA polymerase that acts to increase the processivity of polymerization by similarity. In a preferred embodiment, the sequence is as set forth in serial number NCBI Accession NO: AA073452 or SEQ ID NO: 4.

CMV p 100 is a CMV capsid protein, which is 150 kDa HCMV phosphoprotein and includes the following characteristics: (1) Name: pp150, also named basic phosphoprotein (BPP), and UL32; (2) Organism: Human cytomegalovirus, belonging to Human herpesvirus-5 (HHV-5); (3) Sequence length: 1046 amino acids; and (4) Function: large structural phosphoprotein. In a preferred embodiment, the sequence is as set forth in serial number NCBI Accession NO: AAO73452 or SEQ ID NO: 5.

In a preferred embodiment of the present invention, CMV pp65, IE-1, VGLB, VPAP and CMV p100 polypeptides respectively have a sequence essentially identical to their corresponding sequences as mentioned above.

According to the present invention, the term " -derived peptide fragment" refers to peptide fragment or peptide that has sequences of whole or a portion of protein.

According to the present invention, the term "essentially identical to" refers to the variation of amino acid sequence that is not necessarily affecting the activity of formed protein in various species. Therefore, said variation will not affect the activity of formed protein only if the amino acid sequence has a certain homology among identified sequences. Preferably, said certain homology is more than 70%, more preferably, having 80% of homology, and the most preferably, having 90% of homology.

In a preferred embodiment of the present invention, the above-mentioned immunogenic composition includes CMV pp65, CMV IE-1, CMV VGLB, CMV VPAP and CMV p100 polypeptide -derived peptide pools. The term "peptide pool", as used hereby, refers to a combination containing partially overlapping peptides spanning whole sequence or a portion sequence of a gene product.

In a preferred embodiment of the present invention, each peptide of the polypeptides-derived peptide pool has a length of 12 to 18 amino acids.

In a preferred embodiment of the present invention, wherein the two adjacent peptides of each peptide pool has a 10 to 15 continuous amino acid residues overlapping in sequence.

For example, if the aforesaid polypeptide-derived peptide pool includes pentadecapeptides, that means a peptide pool of 15-mer peptide spanning full-length gene product with partial amino acid overlapping in sequence, and likewise the peptide pools with peptides of other lengths.

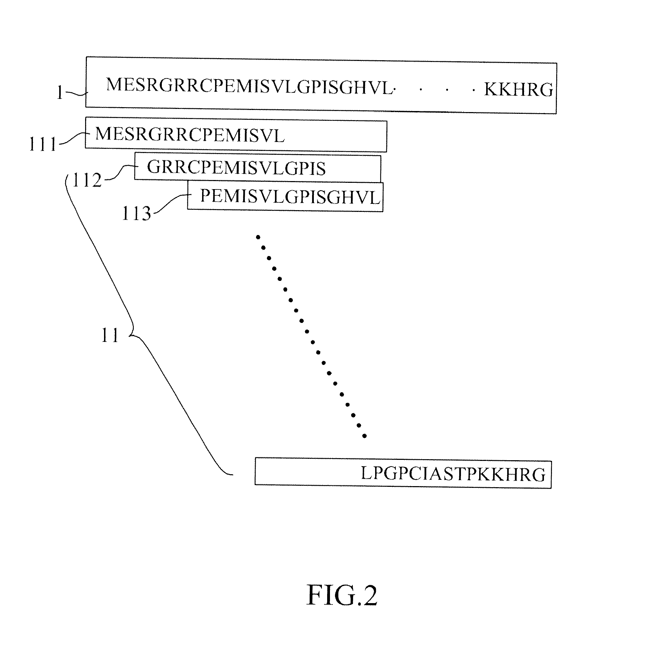

In a preferred embodiment of the present invention, the two adjacent pentadecapeptides in the peptide pool derived from each polypeptide have 11 continuous amino acid residues overlapping in sequence. For instance, as shown in Fig.2, the gene product of CMV pp65 gene has a sequence as set forth in SEQ ID NO: 1, including 561 amino acids. The pentadecapeptides (11) derived from CMV pp65 polypeptide include multiple peptides, wherein a 15-mer peptide (111) has a sequence of 15 continuous amino acids from the site 1^{st} to 15^{th}. Another adjacent 15-mer peptide (112) has a sequence of 15 continuous amino acids from the site 5^{th} to 15^{th}, containing 11 continuous and partially overlapping amino acids, and 4 continuous amino acid residue sequences from the site 16^{th} to 19^{th}. Yet another adjacent 15-mer peptide (113) has a sequence of amino acids from the site 9^{th} to 19^{th}, containing 11 continuous and partially overlapping amino acids and 4 continuous amino acid residues from the site 20^{th} to 23^{rd}, and so on. For the last sequence that has less than four continuous amino acid residues, number of its overlapping amino acids is increased to be pentadecapeptide such that each peptide of the peptide pools is 15-mer.

According to the present invention, the above principle is also adapted to the CMV IE-1, CMV VGLB, CMV VPAP and CMV p100 polypeptide-derived peptide pools.

According to the present invention, said peptide pools are prepared by methods for preparing peptide pools known in the art. In a preferred embodiment of the present invention, said peptide pools are composed of synthetic peptides.

According to the present invention, said adjacent peptides in said peptide pool refer to two peptides having the maximal overlapping continuous amino acid sequences. Take said peptide pool of 15-mer peptides for example, if the peptide pool is set to have 11 continuous amino acid sequences overlapping, the two adjacent peptides respectively have 11 continuous amino acid sequences close to C-terminal and 11 continuous amino acid sequences close to N-terminal, which are identical.

According to the present invention, said immunogenic composition further includes a physiologically acceptable carrier or excipient.

According to the present invention, said immunogenic composition further includes an immunostimulant.

The term "immunostimulant" as used hereby refers to any material, which could substantially improve or enhance external antibody- or cell-mediated immune responses against exogenous antigen. A preferable immunostimulant includes an adjuvant. The adjuvant includes materials which are designed to protect antigen from fast metabolism, such as aluminum hydroxide or mineral oil; and a stimulator of immune responses, such as copolymer of surface active agent, including lipid A. Also included are *Bordetalla pertussis* derived proteins and *Mycobacterium tuberculosis* derived proteins, and glycolipids used as Immunoregulator, such as α-galatosylceramide (α-GalCer) and derivatives, and CqG derived polynucleotides.

Specifically, adjuvants were any commercially available agent such as Freund's Incomplete Adjuvant and Complete Adjuvant (Difco Laboratories, Detroit, Mich.); Merck Adjuvant 65 (Merck and Company, Inc., Rahway, N.J.); AS-2 (SmithKline Beecham, Philadelphia, Pa.); aluminum salt, such as aluminum hydroxide gel or aluminum phosphate; the salt of calcium, iron or zinc; insoluble suspension ofacylated tyrosine; acylated sugars, cationically or anionically derivatized polysaccharides; polyphosphazenes; biodegradable microspheres; monophosphoryl lipid A and *Quillaja saponaria* A (Quil A); and others such as GM-CSF, Interleukin-2, -7, -12 cytokines and other similar growth factors also could be used as adjuvants.

In another aspect, the present invention provides a method for *ex vivo* activating immune cells, comprising steps of: mixing an immune cell and the aforesaid immunogenic composition to form a mixed cell culture; then incubating the mixed culture in a suitable medium to obtain the activated immune cell.

According to the present invention, said immune cell is an antigen-presenting cell; preferably, said immune cells include dendritic cells; preferably dendritic cells are derived from peripheral blood mononuclear cells, bone marrow cells, hematopoietic progenitor cells or stem cells.

As known by persons of ordinary skill in the art, dendritic cells are leukocytes existing in mammals, mainly in blood, exposed tissues and other tissues, such as in epithelia tissue of skin, nasal cavity, lung, stomach and intestine. The functions of dendritic cells are regulating innate and acquired immune responses induced by environmental stimulus; wherein the most important function thereof is to process antigen and then present the processed antigen to other leukocytes of immune system; therefore dendritic cells belong to antigen-presenting cells. (Science, 288: 522-527, 2000; Curr. Med. Chem., 13(14): 1591-607, 2006; Nat. Rev. Cancer, 8(5): 351-60, 2008) In a preferred embodiment of the present invention, the dendritic cells are human dendritic cells.

According to the present invention, said suitable medium comprises cytokines or growth factors; the cytokines are selected from the group consisting of IL-2, IL-7 and combination thereof.

In yet another aspect, the present invention provides a method for *ex vivo* inducing immune effector cells, comprising steps of: providing an aforesaid activated immune cell and a lymphocyte; then co-incubating the activated immune cell and lymphocyte in a suitable medium to obtain an immune effector cell.

Preferably, the lymphocyte is an autologous or allergenic lymphocyte. More preferably, the lymphocyte is an autologous lymphocyte.

According to the present invention, the lymphocytes are derived from non-adherent peripheral blood mononuclear cells. In a preferred embodiment of the present invention, the lymphocyte is T lymphocyte or B lymphocyte; more preferably, the T lymphocyte is an autologous lymphocyte.

The term "autologous" as used hereby and known by persons of ordinary skill in the art, refers to being derived from the same individual. For instance, said autologous immune cells refer to the provided dendritic cells derived from selfsame individual, whereby avoiding unwanted immune response, such as heterologous immune response.

Based on the immunogenic composition of the present invention, which is derived from cytomegalovirus belonging to herpes virus family, the obtained activated immune cell according to the method of the present invention could also activate herpes virus-specific lymphocyte, and then be applied for inducing immune response against herpes virus.

In accordance with the present invention, the herpes virus includes, but is not limited to: cytomegalovirus, Herpes simplex virus-1 (HSV-1), Herpes simplex virus-2 (HSV-2), Varicella Zoster virus (VSV), Epstein-Barr Virus (EBV), Human herpes virus-6 (HHV-6), Human herpes virus-7 (HHV-7) and Human herpes virus-8 (HHV-8).

The present invention was further illustrated by the following examples; it should be understood that the examples and embodiments described herein are for illustrative purposes only and should not be construed as limiting the embodiments set forth herein.

### Materials and Methods:

### 1. Peptides and antigens

The 15-mer peptide pools were purchased from JPT Peptide Technologies GmbH (Berlin, Germany), including the mixtures of pentadecapeptides of 11 partially overlapping amino acids spanning the entire 427 amino acids of the HCMVA pp65 (SEQ ID NO: 1) (561 amino acids, 138 peptides), HCMVA IE-1(SEQ ID NO:2) (491 amino acids, 120 peptides), HCMVA VPAP (SEQ ID NO:3) (433 amino acids, 106 peptides), HCMVA p100 (SEQ ID NO:4) (1048 amino acids, 259 peptides), and HCMVT VGLB (SEQ ID NO:5) (907 amino acids, 224 peptides). Other antigens and peptides can be synthesized by different methods, including WT-1 (Wilm's tumor 1) -derived peptide pool (Swiss prot: P19544; as shown by SEQ ID. NO:6) (WT33; JPT Peptide Technologies GmbH, Berlin, Germany)and core protein of Hepatitis C virus-derived peptide pool (NCBI ACCESSION: ABV46234 (1-191 //product="core protein"; as shown by SEQ ID. NO: 7), and Peptide Scan 15/11, from JPT Peptide Technologies GmbH, Berlin, Germany, all of which are used as non-specific peptide pools.

### 2. Isolation of peripheral blood mononuclear cells (PBMCs)

Apheresis blood or whole blood was voluntarily donated by healthy donors. PBMCs were prepared by gradient density centrifugation in Ficoll-Hypaque (GE Healthcare Bio-Sciences AB, NJ, USA) as previously described {Han, 2008 #5292}. Viability of the human PBMCs was determined by trypan blue staining and only cells with a viability of ≧80% were used.

### 3. Preparation of dendritic cells (DCs)

PBMCs were placed into 6-well plates of 1x10⁷ cells/well and adhered for 2 hours in the medium AIM-V (Gibco-BRL, CA, USA). Then, non-adherent cells were removed gently and frozen as source of T cells for further co-culture. Adherent cells were cultured in the medium AIM-V supplemented with 50 ng/ml GM-CSF (Biosource, CA, USA) and 25 ng/ml IL-4 (Biosource, CA, USA). For the generation of DCs, cells were cultured with GM-CSF and IL-4 for 24 hours and incubated for another 24 hours with IFN-γ (20 ng/ml) (Gentaur), TNF-α (50 ng/ml) (R&D systems, MN, USA), IL-1β (10 ng/ml) (R&D systems, MN, USA), IL-6 (10 ng/ml) (R&D systems) and PGE2 (1 µM) (Sigma-Aldrich, MO, USA) for maturation. The phenotype of DCs was analyzed with flow cytometry.

### 4. Dendritic cell-activated and ex vivo expanded antigen-specific immune cells

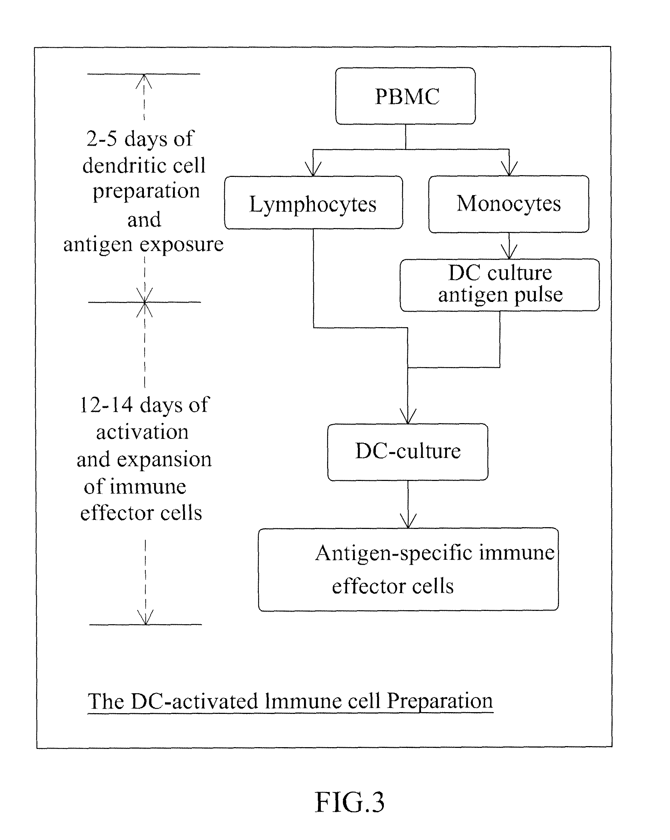

As shown in Fig.3, the methods were approximately processed as shown in the figure. Dendritic cell-activated immune cells were generated as previously described (Han et al, *supra*). In brief, mature DCs were loaded with peptides (5 µg/ml) or other antigens for 3 hours and irradiated (2,500 rads). The DCs were cocultured with autologous non-adherent PBMCs at a ratio of 1:20 in AIM-V with 2% human AB serum. On Day 3, IL-2 (Gentaur, Aachen, Germany), IL-7 (Gentaur) and IL-15 (Gentaur) were added. Then, fresh medium with cytokines were added every other days. The function of T cells was analyzed by intracellular cytokine staining

### 5. CFSE cell proliferating analysis

Briefly, 1x10⁶ PBMCs were labeled with CFSE (carboxyfluorescein diacetate succinimidyl ester-based), and then stimulated by antigens in culture. On Day 3, IL-2 (Gentaur, Aachen, Germany) and IL-15 (Gentaur) were added. Then, fresh medium with cytokines were added every other days until Day 7 and then were subjected to flow cytometry analysis.

### 6. CD107a and intracellular cytokine staining

The assay was performed as described (Han *et al., supra*). Briefly, 3x10⁵ expanded CMV immune effector cells were stimulated with antigen-loaded autologous DCs or monocytes in AIM-V with or without anti-CD 107a-FITC. Monensin (Sigma) was added 1 hour after stimulation. After 5 hours, cells were stained for CD4 and CD8, fixed, permeabilized, and stained with antibodies against IFN-γ (or TNF-α, or IL-2) (all from BD Bioscience) using FIX/PERM and PERM/Wash solution (BD).

### 7. Enzyme-linked ImmunoSpot assay (ELISpot assay)

Interferon-y (IFN-γ) ELISPOT assays were performed by incubation of 1x10⁵ PBMCs/well with different combinations of pepmix in ninety-six-well, ELISPOT plates (Millipore, MA) precoated with anti-human IFN-γ monoclonal antibody (1-D1K; MabTech, Sweden) and blocked by 5% inactivated human AB serum in duplicate. After 18 to 20 hours of culturing at 37°C in a humidified incubator under 5% CO₂ plates were washed and stained with 1 µg/ml biotinylated anti-human IFN-γ as the secondary antibody (7-B6-1; MabTech), followed by streptavidin conjugated alkaline phosphatase (streptavidin conjugated ALP) (MabTech) and BCIP/NBT-plus substrate (Bio-Rad) to develop the color reaction. The colored spots were counted using the EliSpot Reader (AutoImmune Diagnostika). Results were presented by IFN-γ spot-forming cells (SFC)/10⁶ PBMCs.

### Example.1 To analyze the effect of peptide pools of various CMV proteins and their combinations on stimulating PBMC by CFSE staining method.

The objective of the present example is to use peptide pools of various CMV proteins and their combinations to stimulate PBMC, and analyze the effect of peptide pools of various CMV-derived proteins and their combinations on proliferation of PBMC by CFSE staining, to understand the effect of various combinations on inducing immune response.

By method as described in *General materials and method,* CFSE-labeled PBMCs were stimulated by various peptide pools or their combinations for 7 days, as divided into the following groups:
(1) The CMV pentadecapeptides of pp65 were indicated as sample 1.
(2) The CMV pentadecapeptides of VGLB were indicated as sample 2.
(3) The CMV pentadecapeptides of IE-1 were indicated as sample 3.
(4) The CMV pentadecapeptides of p100 were indicated as sample 4.
(5) The CMV pentadecapeptides of VPAP were indicated as sample 5.
(6) The CMV pentadecapeptides of pp65 and VGLB were indicated as sample 6.
(7) The CMV pentadecapeptides of pp65 and IE-1 were indicated as sample 7.
(8) The CMV pentadecapeptides of VGLB and IE-1 were indicated as sample 8.
(9) The CMV pentadecapeptides of pp65, VGLB and IE-1 were indicated as sample 9.
(10) The CMV pentadecapeptides of pp65, VGLB, IE-1 and p100 were indicated as sample 10.
(11) The CMV pentadecapeptides of pp65, VGLB, IE-1, p100 and VPAP were indicated as sample 11.

Further, the following groups worked as the control:
(12) CFSE-labeled PBMCs were indicated as no stimulation control for background value as sample 12.
(13) Co-culture of CFSE-labeled PBMCs and dendritic cells loaded with core protein of Hepatitis C virus derived peptide pool worked as the negative control, indicated as sample 13.

The above stimulated-PBMCs were subjected to antigen-specific proliferation and intracellular cytokine staining analysis by the method as described in *General materials and method.* The obtained results were minus value obtained from PBMCs before being plotted as a graph.

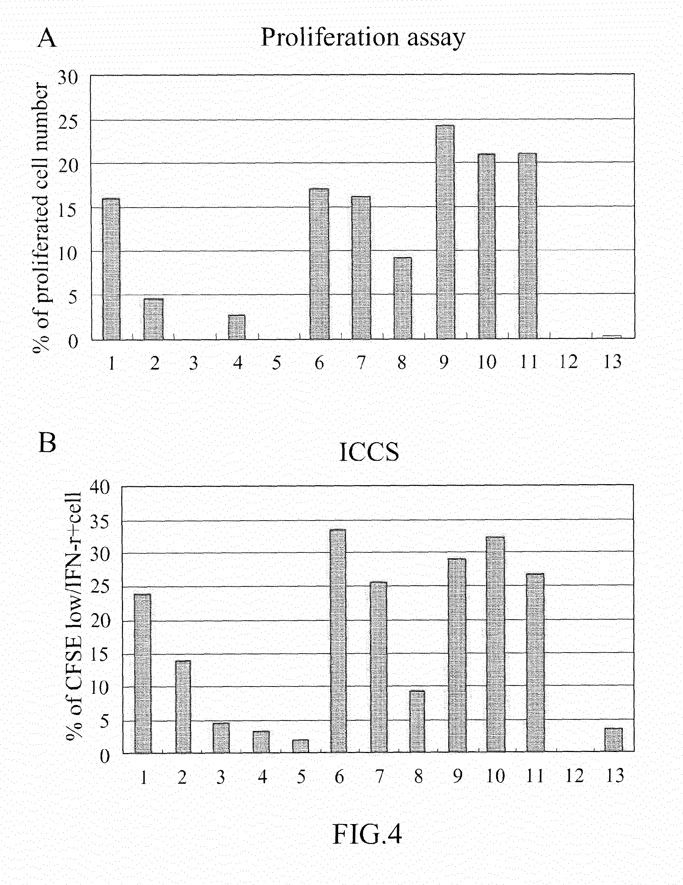

As shown in Fig. 4, panel A illustrates the situation of cell proliferation under the stimulation by various mixed peptide pools; panel B illustrates cells' abilities to express IFN-γ, wherein the cells proliferate under the stimulations by various mixed peptide pools.

The above results show that: (1) 5 CMV proteins-derived peptide pools individually induce responses; (2) any single polypeptide of pp65, VGLB or p100 polypeptide derived peptide pool could also induce immune response; (3) the result of two peptide pools mixed is better than only one peptide pool, and (4) the immune response induced by the peptide pool either of pp65 or VGLB is better than any of the rest CMV protein-derived peptide pool, combination of said peptide pools has additive effect on enhancing immune response, and the effect is better when more peptide pools are mixed.

### Example.2 To analyze the effect of peptide pools of various CMV proteins and their combinations on stimulating PBMC by ELISpot assay.

The objective of the present example is to use various CMV protein-derived peptide pools and their combinations to stimulate PBMC, and analyze the effect of various CMV protein-derived peptide pools and their combinations on cellular function of PBMC by ELISpot assay.

By method as described in *General materials and method,* newly-isolated PBMCs were separately obtained from various donators. PBMCs were further stimulated by the following groups of various combinations of peptide, incubated for 20 hours, and processed with IFN-γ analysis by ELISpot as above-mentioned in *General materials and method.*
(1) The CMV pentadecapeptides of pp65 were indicated as sample 1.
(2) The CMV pentadecapeptides of VGLB were indicated as sample 2.
(3) The CMV pentadecapeptides of IE-1 were indicated as sample 3.
(4) The CMV pentadecapeptides of pp65 and VGLB were indicated as sample 4.
(5) The CMV pentadecapeptides of pp65 and IE-1 were indicated as sample 5.
(6) The CMV pentadecapeptides of pp65, VGLB and IE-1 were indicated as sample 6.
(7) The CMV pentadecapeptides of pp65 VGLB, IE-1 and p100 were indicated as sample 7.
(8) The CMV pentadecapeptides of pp65, VGLB, IE-1, p100 and VPAP were indicated as sample 8.

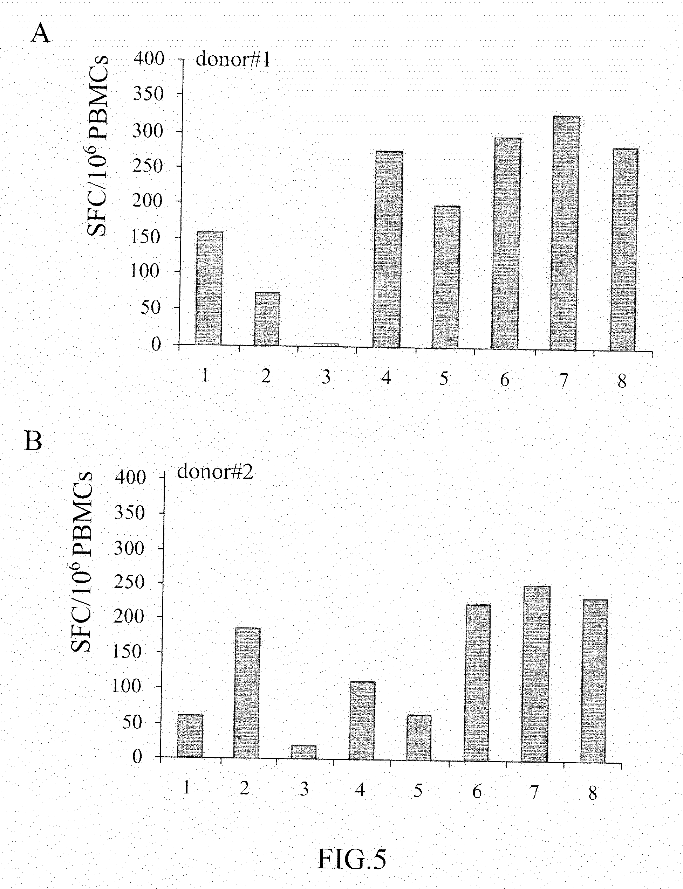

As shown in Fig. 5, the effect of the group pp65+VGLB (as bar 4) or pp65+IE-1 (as bar5) on stimulating PMBC to produce IFN-γ is more prominent than that of pp65 (as bar1). The stimulating effect of pp65+VGLB (bar4) is better than pp65+IE-1 (as bar5). And the effect of combination of various CMV protein -derived peptide pools (as bar 6, 7 or 8) is better than that of the group having only two CMV protein-derived peptide pools (as bar 4 or 5) or that of the group of pp65 alone (as bar 1).

### Example.3 To analyze the proliferation of cell populations of PBMC stimulated by CMV proteins-derived peptide pool and their combinations.

The objective of the present example is to use various CMV protein-derived peptide pools and their combinations to stimulate PBMC, and to analyze the effect of the CMV protein-derived peptide pools and their combinations on inducing proliferation of CD4⁺ and CD8⁺ populations in PBMC.

The CFSE-labeled PBMCs were respectively cultured with the stimulus as indicated as the following: (1) the pentadecapeptides of HCMVA pp65 was indicated as "pp65"; (2) the mixed pool of pentadecapeptides of both HCMVA pp65 and HCMVA IE-1, including HCMVA pp65 polypeptide-derived peptide pool and HCMVA IE-1 polypeptide-derived peptide pool, were indicated as "pp65/IE-1"; (3) the mixed pool of the pentadecapeptides of HCMVA pp65, HCMVA IE-1, HCMVA VPAP, HCMVA p100 and HCMVT VGLB were indicated as "5 pepmix"; (4) the nonspecific pentadecapeptides were indicated as "WT-1"; (5) anti- CD3 antibody as positive control was indicated as ''anti-CD3", and, incubated for seven days, and were subjected to antigen-specific proliferation by the method as mentioned in *General materials and method,* especially to cell proliferation of CD4⁺ and CD8⁺ cell populations, and separately incubated CFSE-labeled PBMC as no stimulation control was indicated as "PBMC".

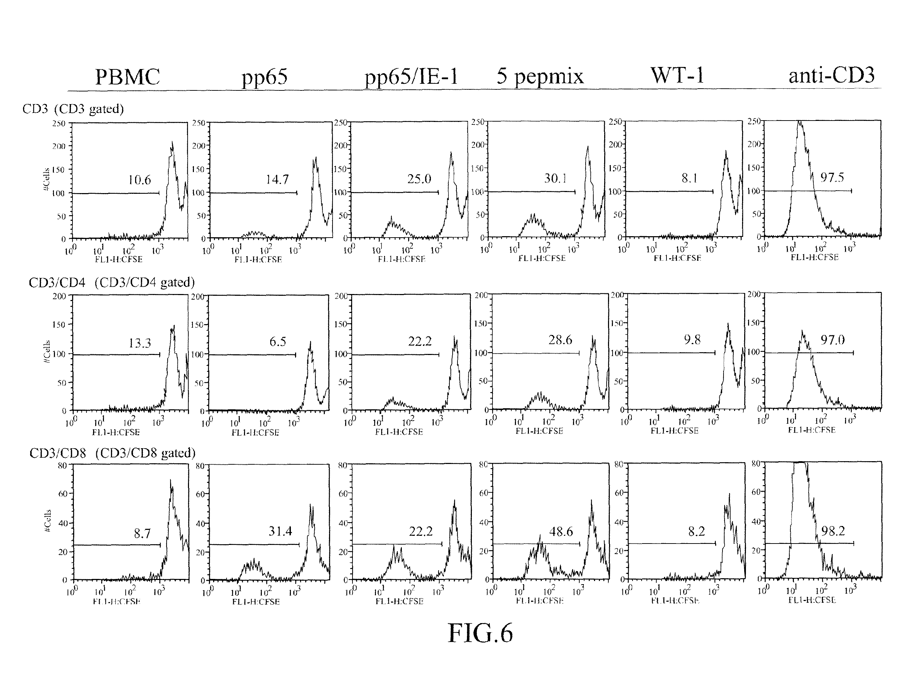

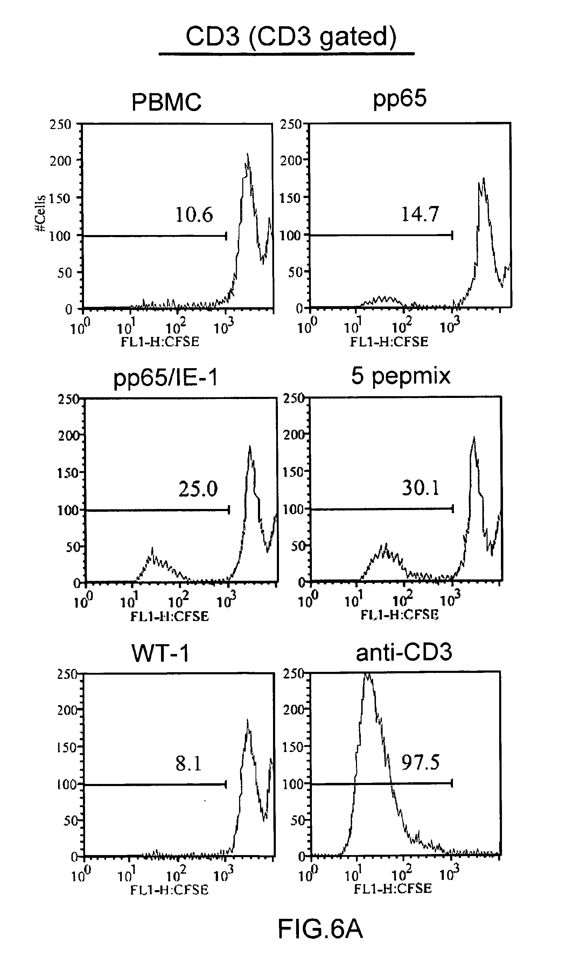

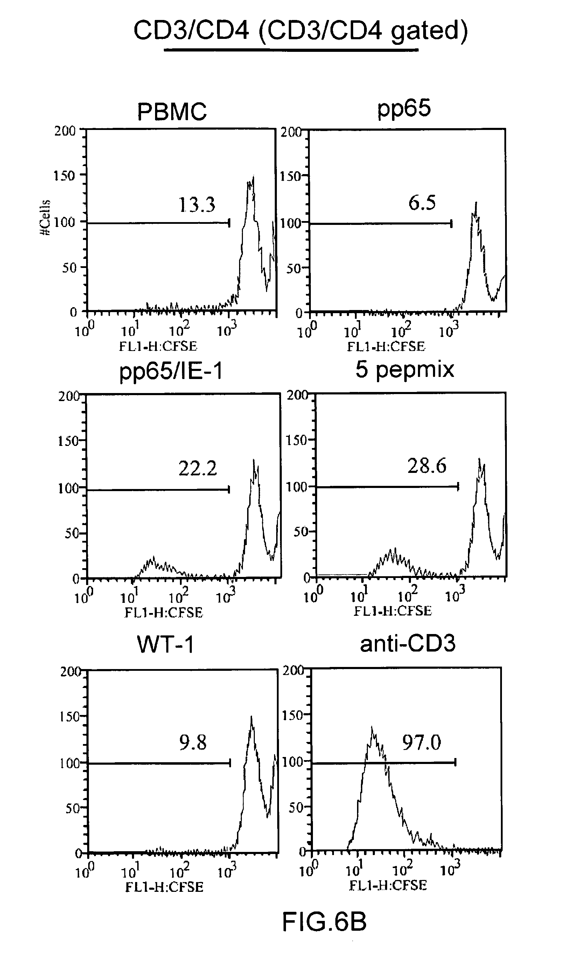

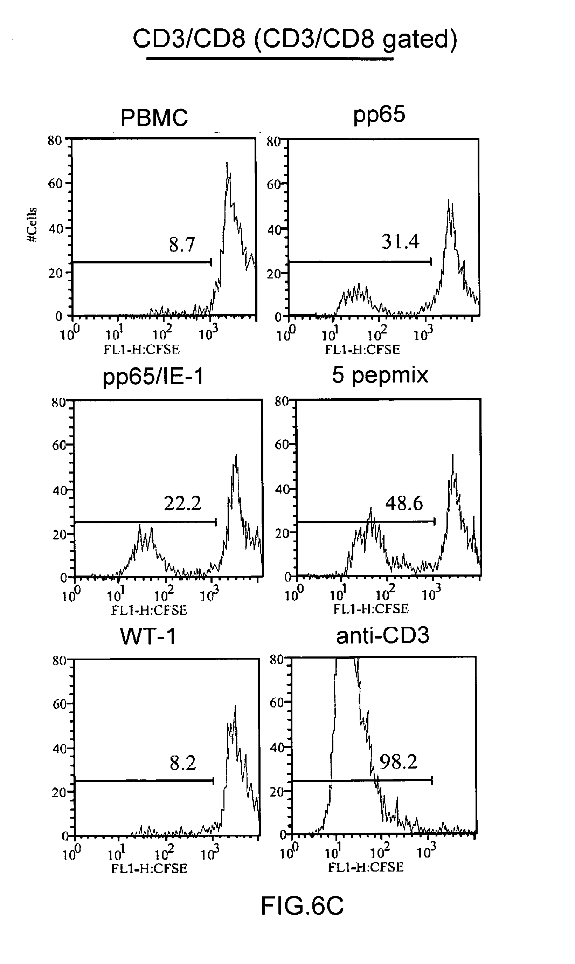

As shown in Fig. 6, the percentage of CFSE-diluted cells (i.e. proliferation cell) of each group was as indicated. Among the mixed pool of CMV protein-derived pentadecapeptides (pp65) or the mixed pool of two CMV-derived protein pentadecapeptides (pp65/IE-1), the mixed pool of pentadecapeptides derived from five CMV protein (5 pepmix) induced the highest proliferation of CD4⁺ and CD8⁺ groups cell populations within PBMC, demonstrating that the mixture of peptide pools derived from five CMV protein had the best immunogenicity.

### Example.4 Functional analysis of various peptide pools derived from CMV protein stimulating PBMC.

The present invention further analyzed the effect of CMV protein-derived peptide pools and their combinations on stimulating the cells within PBMC, and analyzed cytokinessecretion of PBMC to determine immune function and confirm the immunogenicity of various combinations of peptide pools.

The PBMCs from two donators were respectively stimulated by the stimulus as follows: (1) the pentadecapeptides of HCMVApp65 were indicated as "pp65";(2) the mixed pool of pentadecapeptides of both HCMVA pp65 and HCMVA IE-1, including HCMVA pp65 polypeptide-derived peptide pool and HCMVA IE-1 polypeptide-derived peptide pool, was indicated as "pp65/IE-1"; (3) the mixed pool of pentadecapeptides of HCMVA pp65, HCMVA IE-1, HCMVA VPAP, HCMVA p100 and HCMVT VGLB was indicated as "5 pepmix"; (4) the nonspecific pentadecapeptides were indicated as "WT-1"; (5) the anti- CD3 antibody as positive control was indicated as "anti-CD3", and was incubated for seven days and processed with intracellular cytokine staining( ICCS) as mentioned in *General materials and method,* to analyze expression of IL-2 and IFN-γ. Separately incubated PBMC was used as no stimulation control and indicated as "PBMC only".

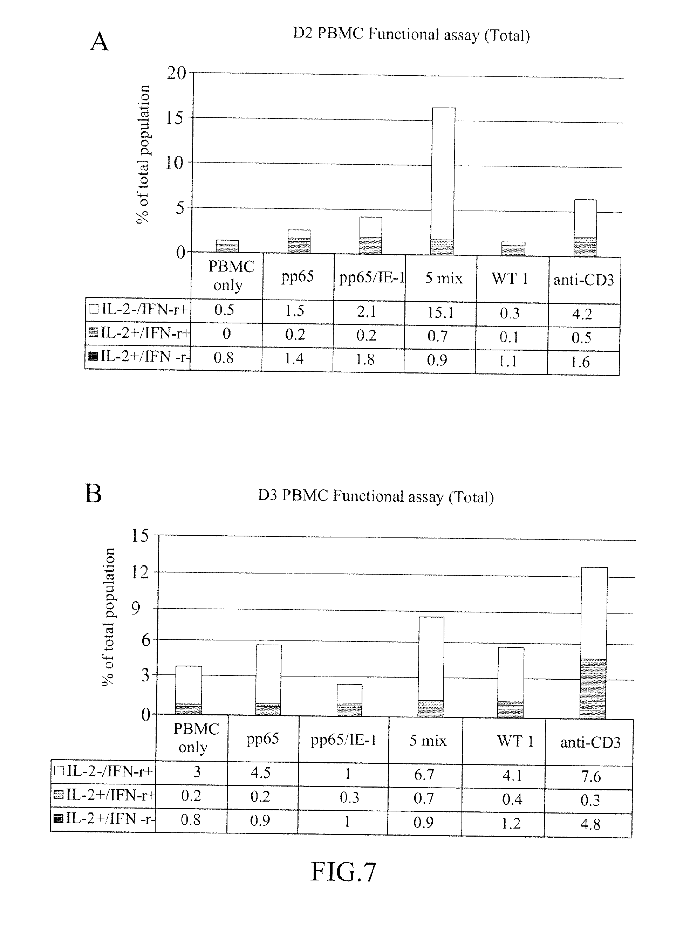

Fig.7, panels A and B respectively expressed results of PMBCs from various origins by the above-mentioned analysis, as illustrated by the bar chart, respectively representing expression of IL-2 and/or IFN-γ of PBMC. Based on the results, among the peptide pool derived from single CMV protein (pp65) or the mixed pool of peptide pool derived from two CMV proteins (pp65lIE-1), the mixed pool of peptide pools of five CMV antigen (5 pepmix) stimulated had a highest activity to stimulate PBMCs to express IL-2 and/ or IFN-γ, demonstrating that the combination of peptide pools of five CMV proteins had the best immunogenicity.

### Example.5 To produce CMV-specific immune cells by stimulating PMBC with dendritic cells (DCs).

The present example used DCs that were pulsed with the mixed pool of pentadecapeptides derived from five CMV proteins to stimulate non-adherent PBMC to evaluate its effect on generation of CMV-specific immune cells.

The dendritic cells treated by the mixed pool of pentadecapeptides derived from 5 CMV proteins and non-adherent PMBC (as the source of T lymphocytes) were prepared by the method as mentioned in *General materials and method,* and co-incubated (as shown in Fig.3). After being co-incubated for 15 days, the DC-activated lymphocytes were respectively re-stimulated by the dendritic cells loaded with an individual peptide pool derived from each CMV protein (pp65, IE-1, VGLB, VPAP and p100) and their combination for five hours, meanwhile Phorbol (PMA) and Ionomycin (PMA+Ionomycin) were used to stimulate DC-activated lymphocytes as positive control and homologous dendritic cell treated with non-specific antigen was used as control. For evaluating the function of antigen specific-immune cell, the re-stimulated lymphocytes were examined by intracellular cytokine staining (ICCS) to IFN-γ, IL-2 and TNF-α, and by the staining the cell marker of degranulation CD107a.

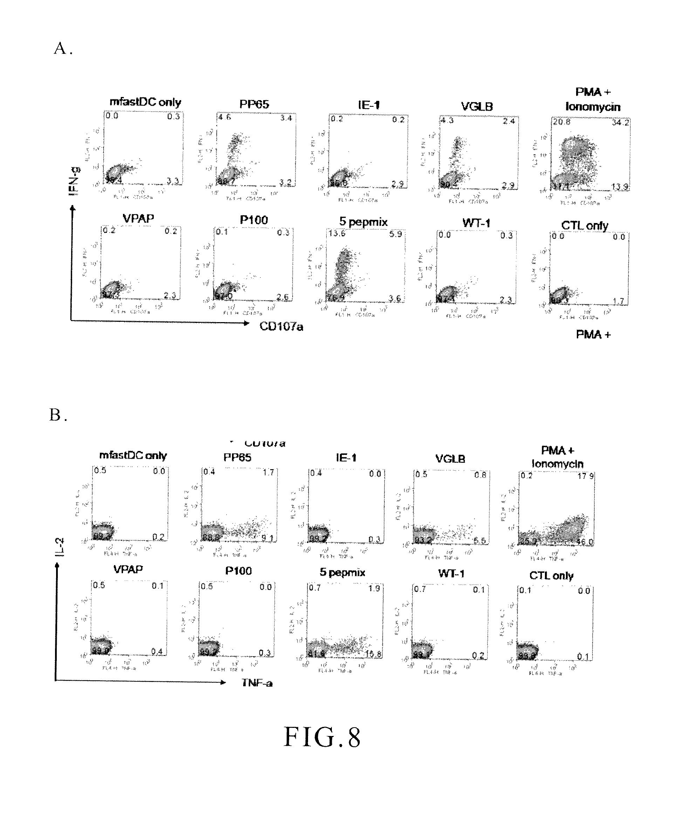

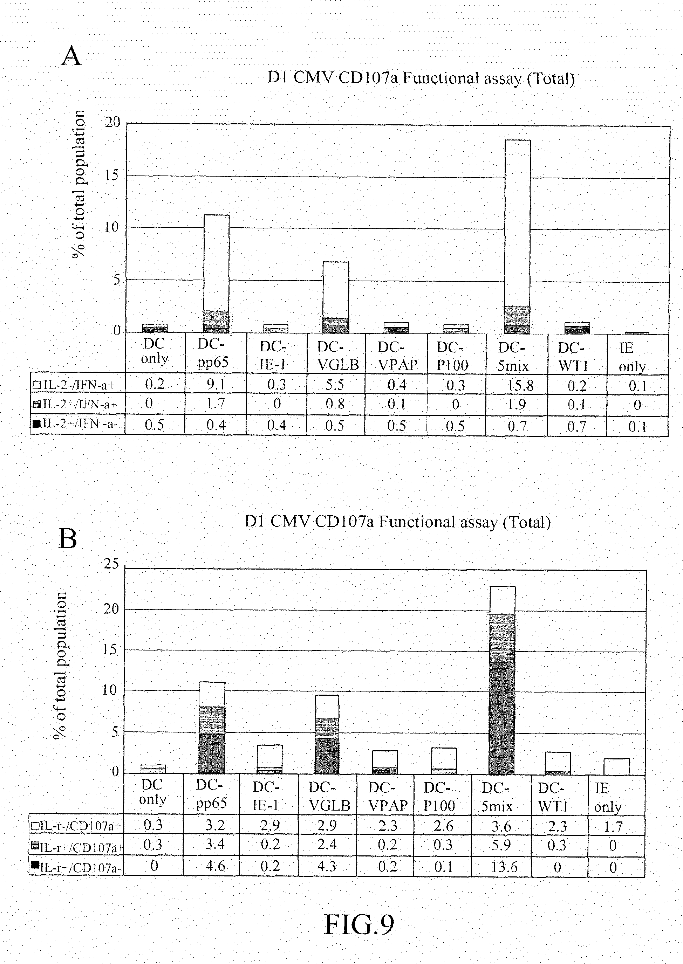

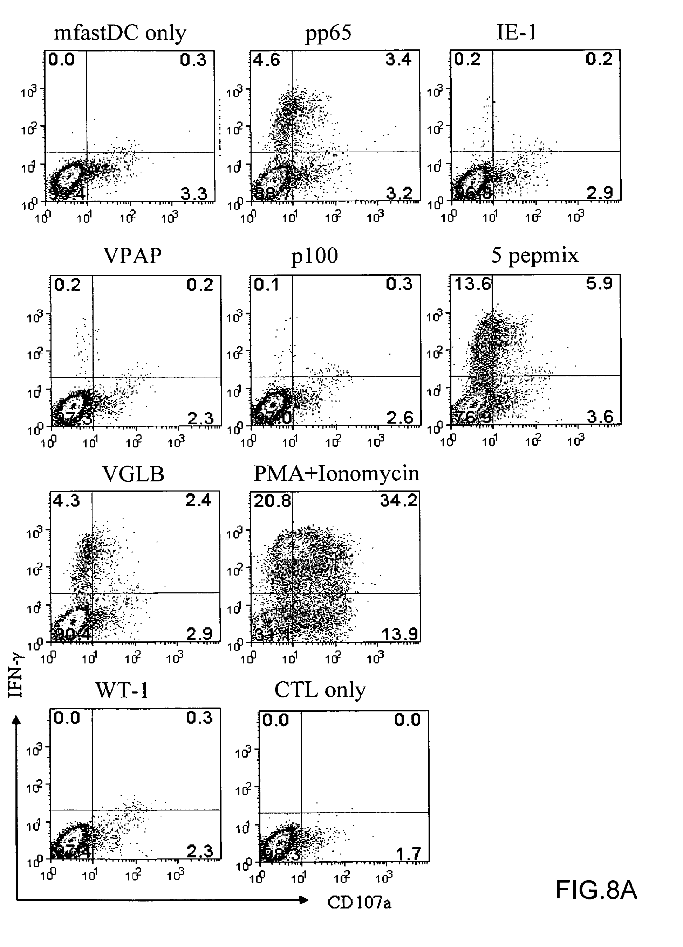

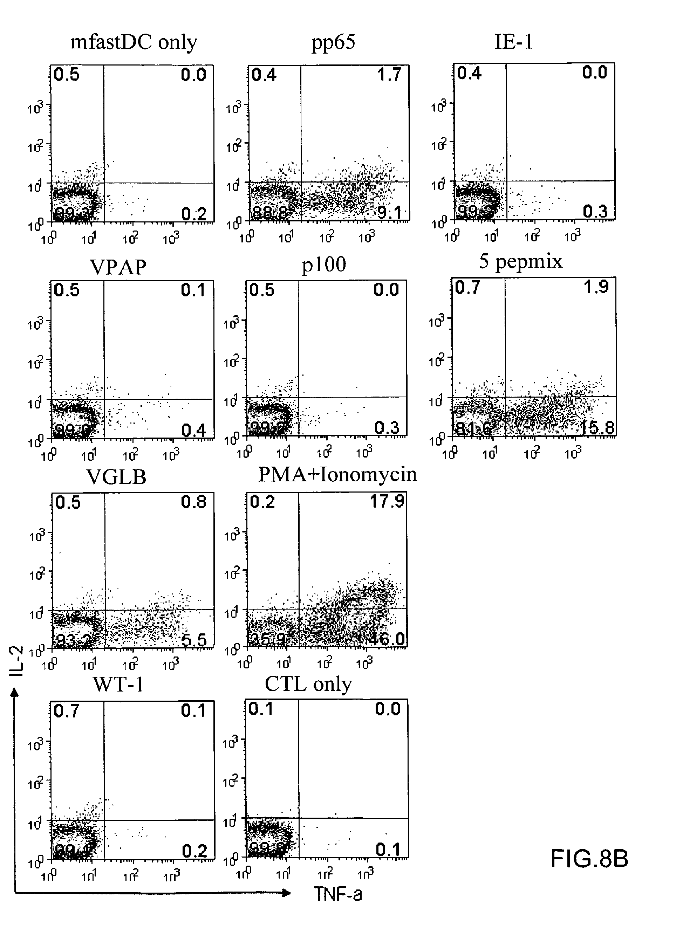

Fig.8 was the result of the above intracellular cytokine staining, wherein the expression of cytokine such as IFN-γ, IL-2, TNF-α, and CD107a was as indicated. The bar chart illustrated in panel A and panel B of Fig.9 was plotted based on the result of Fig.8, and respectively demonstrated the percentages of cell populations expressing IL-2 and/ or TNF-α, and the percentages of cell populations expressing IFN-γ and/or CD107a. The result showed that activated-immune cells induced by 5 pepmix had specific response to the pentadecapeptides derived from any of CMV proteins, wherein the immune response against pp65 and VGLB was the most prominent one. The group that is re-stimulated group by dendritic cells treated with 5 pepmix induced a highest cytokine production of immune cells.

The result of the above examples demonstrated that the combined pools of pentadecapeptides derived from multiple CMV proteins (up to five proteins) could effectively activate both CD4⁺ and CD8⁺ T lymphocytes in a short period of time. The antigen-specific proliferation and the production of effector cytokine of CD4⁺ and CD8⁺ T lymphocytes were improved obviously, demonstrating that the combination of pentadecapeptides derived from CMV proteins was useful as an effective vaccine or immune treatment for establishing anti-CMV immunity. As shown in Fig.6 and Fig.7, the combination of pentadecapeptides derived from five CMV proteins induced proliferation and cytokine production from CD4⁺ and CD8⁺ cells in PBMC. Although pentadecapeptides derived from single or two CMV proteins could stimulate immune response, the combination of pentadecapeptides derived from five CMV proteins could generate the strongest antigen specific immune response. The function of immune effector cells was analyzed and assessed by multi-color flow cytometry staining immune effector cell cytokines and cell marker of degranulation, CD107a. As shown in Fig.8 and Fig.9, compared with pentadecapeptides derived from only one or two CMV proteins, the combination ofpentadecapeptides derived from five CMV proteins (5 pepmix) is the most effective to induce production of immune effector cytokine and cell marker of degranulation, of CD 107a, with the combination of the five CMV peptide pools being the most effective CMV vaccine or immunogen.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. A method for activating immune cells **characterized in** comprising steps of:
mixing an immune cell and an immunogenic composition to a mixed culture; then incubating the mixed cell culture in a suitable medium to obtain the activated immune cell; wherein the immunogenic composition containing CMV-derived peptide comprises:
(a) at least one peptide selected from CMV pp65 and CMV IE-1 polypeptides-derived peptide fragments; and
(b) one or more peptides selected from the group consisting of CMV VGLB, CMV VPAP and CMVp100 polypeptides-derived peptide fragments;
or comprises:
(c) at least one peptide pool selected from CMV pp65 and CMV IE-1 polypeptides -deprived peptide pools; and
(d) one or more peptide pools selected from the group consisting of CMV VGLB, CMV VPAP and CMVp100 polypeptides-derived peptide pools;
or comprises:
(e) a peptide of CMV VGLB polypeptides-derived peptide fragments.

2. The method according to claim 1, wherein the CMV pp65 has a sequence essentially identical to the sequence as set forth in (SEQ ID NO: 1; the CMV IE-1 has a sequence essentially identical to the sequence as set forth in SEQ ID NO; 2; the CMV VGLB has a sequence essentially identical to the sequence as set forth in SEQ ID NO: 3; the CMV VPAP has a sequence essentially identical to the sequence as set forth in SEQ ID NO: 4; and the CMV p100 has a sequence essentially identical to the sequence as set forth in SEQ ID NO: 5.

3. The method according to claim 1, wherein the immunogenic composition comprises CMV pp65, CMV IE-1, CMV VGLB, CMV VPAP and CMVp100 polypeptide-derived peptide pools.

4. The method according to any of claims 1 to 3, wherein the immunogenic composition of each polypeptide-derived peptide pool contains peptides having a length of 12 to 18 amino acids.

5. The method according to claim 4, wherein two adjacent peptides of each peptide pool of the immunogenic composition has 10 to 15 continuous amino acid residues overlapping in sequence.

6. The method according to claim 5, wherein each polypeptide-derived peptide pool of the immunogenic composition includes pentadecapeptides.

7. The method according to claim 6, wherein the two adjacent pentadecapeptides in each polypeptide -derived peptide pool has 11 continuous amino acid residues overlapping in sequence.

8. The method according to any of claims 1 to 3, wherein the immunogenic composition further includes an immunostimulant.

9. The method according to any of claims 1 to 3, wherein the immune cells are derived from peripheral blood mononuclear cells, bone marrow cells, hematopoietic progenitor cells or dendritic cells of stem cells.

10. The method according to any of claims 1 to 3, wherein the suitable medium comprises cytokines; the cytokines are selected from the group consisting of IL-2, IL-7, IL-15 and a combination thereof.

11. A method for inducing production of immune effector cells **characterized in** comprising steps of:
providing an activated immune cell as claimed in any of claims 1 to 3 and a lymphocyte; and
co-incubating the activated immune cell and the lymphocyte in a suitable medium to obtain an immune effector cell.

12. The method according to claim 11, wherein the lymphocyte is derived from non-adherent peripheral blood mononuclear cells.

13. The method according to claim 12, wherein the lymphocyte is T lymphocyte or B lymphocyte.

14. A method for inducing production of immune effector cells **characterized in** comprising steps of:
providing an activated immune cell as claimed in claim 4 and a lymphocyte; and
co-incubating the activated immune cell and the lymphocyte in a suitable medium to obtain an immune effector cell.

15. The method according to claim 14, wherein the lymphocyte is derived from non-adherent peripheral blood mononuclear cells.

16. The method according to claim 14, wherein the lymphocyte is T lymphocyte or B lymphocyte.
